# EUROPÄISCHE PATENTSCHRIFT

(11) **EP 1 068 168 B1**
(45) Veröffentlichungstag und Bekanntmachung des Hinweises auf die Patenterteilung: **28.05.2003**
(21) Anmeldenummer: 99911747.6
(22) Anmeldetag: 08.03.1999
(51) Int. Cl.: C07C 17/16, C07C 19/01

(54) **KONTINUIERLICHES VERFAHREN ZUR HERSTELLUNG VON PROPARGYLCHLORID**
CONTINUOUS METHOD FOR PRODUCING PROPARGYL CHLORIDE
PROCEDE DE PRODUCTION EN CONTINU DE CHLORURE DE PROPARGYLE

(30) Priorität: 09.03.1998 DE 19810036
(43) Veröffentlichungstag der Anmeldung: 17.01.2001
(73) Patentinhaber: BASF AKTIENGESELLSCHAFT, 67056 Ludwigshafen (DE)
(72) Erfinder: STAMM, Armin, D-55128 Mainz (DE); HENKELMANN, Jochem, D-68165 Mannheim (DE); WEYER, Hans-Jürgen, D-67240 Bobenheim-Roxheim (DE)
(74) Vertreter: Isenbruck, Günter, Dr.
(86) Internationale Anmeldenummer: EP9901462
(87) Internationale Veröffentlichungsnummer: WO99046226

(56) Entgegenhaltungen:
- EP-A- 0 514 683
- EP-A- 0 645 357

## Beschreibung

Die Erfindung betrifft ein kontinuierliches Verfahren zur Herstellung von Propargylchlorid durch Umsetzung von Propargylalkohol mit einem Chlorierungsmittel in Gegenwart eines Katalysators.

Propargylchlorid (Propin-3-Chlorid) ist ein wertvolles Zwischenprodukt, daß zur Herstellung einer Reihe von Zwischenprodukten und Galvanohilfsmitteln, aber auch als Einfühnmgsreagenz eines Propargylrestes bei der Herstellung von Pharma- und Pflanzenschutzwirkstoffen benötigt wird.

Es sind zahlreiche Verfahren bekannt, um diskontinuierlich Propargylchlorid aus Propargylalkohol (Propin-3-ol) herzustellen. Im technischen Maßstab ist vor allem die Umsetzung mit Thionylchlorid (SOCl₂) oder Phosgen (COCl₂) geeignet, da als Koppelprodukte nur gasförmige Produkte (SO₂ beziehungsweise CO₂) entstehen, die von selbst aus dem Reaktionsgemisch abdampfen. Phosgen ist besonders bevorzugt, da das entstehende CO₂ im Gegensatz zu SO₂ weniger giftig und weniger umweltschädlich ist. Die Umsetzung von Alkoholen zum Alkylchlorid mit Hilfe von Phosgen erfordert den Einsatz von Katalysatoren.

In der EP-A-0 786 442 ist ein diskontinuierliches zweistufiges Verfahren zur Herstellung von Alkylchloriden mit Phosgen beschrieben, bei dem Hexaalkylguanidiniumhalogenide, substituierte Ammoniumchloride, Phosphoniumhalogenide oder Alkylpyridine als Katalysatoren eingesetzt werden. Nach einer Umsetzung des Alkohols mit Chlorwasserstoff folgt eine Umsetzung mit Phosgen in Gegenwart des Katalysators. Die Temperatur bei der Umsetzung beträgt 120 bis 140°C.

In der DE-A-38 40 340 ist ein Verfahren zur Herstellung von Alkylchloriden beschrieben, bei dem zunächst aus Alkohol und Phosgen das Chlorformiat hergestellt wird, das anschließend nach Zugabe eines Katalysators zum Alkylchlorid decarboxyliert wird. Die Decarboxylierung wird in Gegenwart von quartären Ammonium- oder Phosphoniumsalzen oder ternären Sulfoniumsalzen als Katalysator durchgeführt.

Gemäß EP-A-0 514 683 werden Alkylchloride aus den entsprechenden Alkoholen durch Umsetzung mit Phosgen oder Thionylchlorid in Gegenwart eines aliphatischen, cycloaliphatischen oder cyclisch-aliphatischen Phosphinoxids als Katalysator hergestellt. Die Umsetzung erfolgt bei Temperaturen im Bereich von 84 bis 100°C.

In der EP-A-0 645 357 ist ein Verfahren zur Herstellung von sekundären Alkylchloriden aus den entsprechenden Alkoholen beschrieben, bei dem zunächst das Hydrochlorid von Dimethylformamid durch Einleiten von Chlorwasserstoff hergestellt wird. Sodann wird zur Bildung eines Katalysatoradduktes Phosgen zudosiert. Der so beladene Katalysator wird mit equimolaren Mengen an Alkohol und Phosgen umgesetzt. Der Katalysator wird anschließend abgetrennt und erneut beladen.

Die bekannten Verfahren weisen insbesondere bei der Herstellung von Propargylchlorid eine Reihe von Nachteilen auf. Das zweistufige Verfahren gemäß EP-A-0 786 442 ist aufwendig durchzuführen. Die Decarboxylierung gemäß DE-A 38 40 340 ist insbesondere bei technischen Produktionsmengen aus sicherheitstechnischen Gründen bedenklich. Eine Umsetzung in dem in EP-A-0 514 683 angegebenen hohen Temperaturbereich ist für Propargylchlorid wegen seiner Deflagrationsfähigkeit aufgrund des hohen Energieinhaltes sicherheitstechnisch bedenklich. Das Verfahren gemäß EP-A-0 645 357 erfordert das Vorliegen großer Mengen an beladenem Katalysator. Bei der Beladung des Katalysators entstehen zudem große Mengen an Salzsäure, die nach der Bildung des Propargylchlorides an dieses Addieren und Dichlorpropene bilden können.

Für Pharma- und Pflanzenschutzwirkstoffe wird Propargylchlorid mit einer hohen Reinheit und insbesondere einem sehr niedrigen Gehalt an 1,3- und 2,3-Dichlorpropen benötigt, da diese Nebenprodukte destillativ nur sehr schwer von Propargylchlorid abtrennbar sind und die Reinheit hergestellter Wirkstoffe beeinträchtigen. Zudem müssen entstandene Dichlorpropene im Falle ihrer Abtrennung aufwendig entsorgt werden.

Aufgabe der vorliegenden Erfindung ist die Bereitstellung eines Verfahrens zur Herstellung von Propargylchlorid durch Umsetzung von Propargylalkohol mit einem Chlorierungsmittel in Gegenwart eines Katalysators, das die Nachteile der bekannten Verfahren vermeidet und in hohen Ausbeuten zu Propargylchlorid führt, wobei der Anteil an Dichlorpropenen im Reaktionsaustrag stark vermindert ist. Vorzugsweise soll der Anteil an Dichlorpropenen, bezogen auf die Mengen an erhaltenem Propargylchlorid, weniger als 0,2 Gew.-% betragen.

Die Aufgabe wird erfindungsgemäß durch ein kontinuierliches Verfahren gelöst, bei dem Chlorierungsmittel, Propargylalkohol und 0,1 bis 10 mol.-% des Katalysators, bezogen auf die Menge an Propargylalkohol, kontinuierlich in eine Reaktionszone dosiert und bei einer Temperatur im Bereich von 40 bis 70°C umgesetzt werden.

Das erfindungsgemäße Verfahren erlaubt die Herstellung von Propargylchlorid in hoher Reinheit, wobei der Anteil an als Nebenprodukt gebildeten Dichlorpropenen weniger als 0,2 Gew.-% beträgt. Da das Propargylchlorid aus sicherheitstechnischen Gründen meist nicht mehr destillativ gereinigt wird, wird durch die Verminderung des Nebeqproduktanteils auch die Produktqualität erheblich verbessert.

Die Menge an Katalysator wird so gewählt, daß die gleichzeitige Bildung von Propargylchlorformiat bei der Umsetzung unterbunden wird. Die Chlorformiate können spontan unter Kohlendioxidabspaltung zum Chlorid abreagieren. Da die Decarboxylierung unter erheblicher Freisetzung von Energie und oft unkontrollierbarem Aufschäumen der Reaktionslösung erfolgt, wird die Umsetzung so durchgeführt, daß eine gleichmäßige und vollständige Chlorierung ohne Chlorformiatbildung erfolgt Dies wird durch das Einhalten der vorstehenden Umsetzungsbedingungen, insbesondere der angegebenen Menge an Katalysator und der angegebenen Umsetzungstemperatur, erreicht.

Der Katalysator liegt vorzugsweise in einer Menge von 1 bis 5 mol-% und besonders bevorzugt von 2 bis 3,5 mol-%, bezogen auf die Menge an Propargylalkohol, vor.

Die Umsetzungstemperatur liegt vorzugsweise im Bereich von 50 bis 60°C.

Dabei kann jedes geeignete Chlorierungsmittel eingesetzt werden. Beispiele geeigneter Chlorierungsmittel sind Phosgen und Thionylchlorid. Vorzugsweise wird als Chlorierungsmittel Phosgen eingesetzt

Als Katalysator für die Umsetzung können alle geeigneten Katalysatoren eingesetzt werden. Vorzugsweise ist der Katalysator ausgewählt aus Phosphinoxiden, Guanidiniumsalzen, offenkettigen und cyclischen Alkylharnstoffen, Alkylacetamiden und N,N-Dialkylformamiden sowie Gemischen davon.

Besonders bevorzugt wird als Katalysator mindestens ein Formamid der allgemeinen Formel I 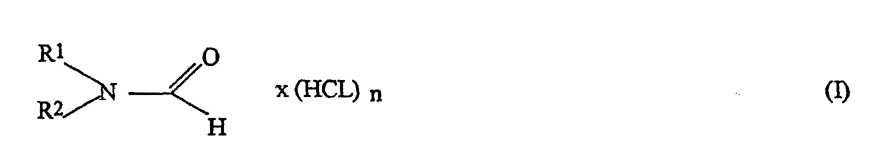 in der R¹ und R² unabhängig voneinander geradkettige oder verzweigte C₁₋₈-, vorzugsweise C₂₋₆-, besonders bevorzugt C₃₋₅-Alkylreste oder gemeinsam einen geradkettigen C₄₋₅-Alkylenrest bedeuten, die jeweils durch 1 bis 4, vorzugsweise 1 oder 2 Sauerstoff- oder Stickstoffatome unterbrochen sein können, und n einen mittleren Wert von 0 bis 3, bevorzugt 0,5 bis 2,5, insbesondere 1 bis 2 hat.

n kann dabei einen Mittelwert für den eingesetzten Katalysator darstellen. Liegt ein genau definierter Gehalt an HCl vor, so ist n vorzugsweise eine ganze Zahl von 0 bis 3.

Dabei wird in der Regel das reine Formamid in das Reaktionsgemisch gegeben, in dem sich während der Phosgenierung das HCl-Addukt bildet.

Bevorzugt sind R¹ und R² die vorstehend beschriebenen geradkettigen oder verzweigten C₁₋₈-Alkylreste. Besonders bevorzugt sind R¹ und R² gleiche Reste, die ausgewählt sind aus n-Butyl, Isobutyl.

Besonders bevorzugt wird als Katalysator Diisobutylformamid eingesetzt.

Die Umsetzung kann in Gegenwart eines inerten Verdünnungsmittels durchgeführt werden. Dabei wird das Verdünnungsmittel vorzugsweise in einer Menge von 10 bis 50 Gew.-%, besonders bevorzugt 20 bis 40 Gew.-%, insbesondere 20 bis 30 Gew.-%, bezogen auf die eingesetzte Menge an Propalgylalkohol, eingesetzt. Vorzugsweise wird als Verdünnungsmittel ein substituierter aromatischer Kohlenwasserstoff oder ein entsprechendes Kohlenwasserstoffgemisch eingesetzt. Besonders bevorzugte Beispiele sind Toluol und Xylol-Isomerengemische sowie Gemische davon.

Die Umsetzung kann in jeder geeigneten Reaktionszone durchgeführt werden. Vorzugsweise wird die Umsetzung in einer Apparatur durchgeführt, die einen Hauptreaktor mit nachgeschaltetem Nachreaktor, der mit einer Strippkolonne zum Strippen von nicht umgesetztem Chlorierungsmittel und gegebenenfalls Propargylchlorid versehen ist, aufweist, wobei die Dosierung der Reaktanten in den Hauptreaktor erfolgt, das Produkt aus dem Nachreaktor ausgeschleust wird und das abgestrippte Chlorierungsmittel und gegebenenfalls Propargylchlorid nach einer Kondensation in den Haupt- und/oder Nachreaktor zurückgeführt wird. Das Phosgen wird gasförmig oder flüssig in das Reaktionsgemisch eingeleitet. Die Temperatur wird im Haupt- und im Nachreaktor im vorstehend angegebenen Bereich gehalten. Das ausgeschleuste Produkt wird in der Regel ohne weitere Reinigung für weitere Umsetzungen eingesetzt.

Durch das erfindungsgemäße kontinuierliche Verfahren kann der Gehalt an Dichlorpropenen, insbesondere 1,3- und 2,3-Dichlorprapen im Reaktionsaustrag auf weniger als 0,2 GC-Flächen- % vermindert werden.

Dabei wird nicht nur der Gehalt an Dichlorpropenen abgesenkt, sondern auch die Farbe des Reaktionsaustrages im Vergleich zu bekannten Verfahren stark aufgehellt. Der verminderte Anteil an farbgebenden Komponenten ist für nachfolgende Umsetzungen von Vorteil, da der Reaktionsaustrag in der Regel nicht weiter aufgearbeitet wird.

Die Erfindung wird nachstehend anhand von Beispielen näher erläutert.

### Beispiele

Die kontinuierlichen Synthesen wurden in einer Apparatur aus einem Haupt- und Nachreaktor sowie einer Strippkolonne durchgeführt. Überschüssiges Phosgen wurde aus dem Nachreaktor ausgetragen, kondensiert und in die Reaktion zurückgeführt. Der Strippkolonne war ein Kondensationskühler nachgeschaltet, der Phosgen und ausgestripptes Propargylchlorid kondensierte, die zusammen in den Nachreaktor zurückgeführt wurden.

### Beispiel 1

In eine Vorlage von 205 g Propargylchlorid und 80 g Toluol wurden stündlich 0,67 Mol (37,5 g) Propargylalkohol, 23,3 g Toluol und 0,023 Mol (3,6 g) Isobutylformamid (3,5 mol-%) so zudosiert, daß die Reaktionstemperatur im Hauptund Nachreaktor bei 50°C gehalten werden konnte. Die Reaktion wurde für insgesamt 24,5 Stunden durchgeführt. Der nach 17,5 bis 24,5 Stunden genommene Reaktionsaustrag enthielt nach der Destillation 60,1% Propargylchlorid (321 g) und - bestimmt mittels GC- 0,12 Flächen-% 1,3-Dichlorpropen und 0,05 Flächen-% 2,3-Dichlorpropen. Der Austrag enthielt weder Propargylchlorfomiat noch unumgesetzten Propargylalkohol.

### Beispiel 2

In eine Vorlage aus dem Reaktionsaustrag aus Beispiel 1 werden pro Stunde 0,67 mol (37,5 g) Propargylalkohol, 23,3 g Toluol und 0,0134 mol (2,1 g) Diisobutylformamid (=2 mol-%) so zudosiert, daß die Reaktionstemperatur in Haupt- und Nebenreaktor bei 50°C gehalten werden kann. Die Reaktion läuft für insgesamt 19,5 h. Der Reaktionsaustrag enthält nach GC-Analytik 63% Propargylchlorid, 0,10 % 1,3-Dichlorpropen und 0,04 % 2,3-Dichlorpropen, kein Chlorformiat und keinen unumgesetzten Propargylalkohol.

### Beispiel 3

In den Reaktionsaustrag aus Beispiel 2 werden pro Stunde 1 mol (56 g) Propargylalkohol, 35 g Toluol und 0,0198 mol (3,1 g) Diisobutylformamid (=2 mol-%) so zudosiert, daß die Reaktionstemperatur in Haupt- und Nebenreaktor bei 50°C gehalten werden kann. Die Reaktion läuft für insgesamt 10 h. Der Reaktionsaustrag enthält nach GC-Analytik 65% Propargylchlorid, 0,06 % 1,3-Dichlorpropen und 0,03 % 2,3-Dichlorpropen, kein Chlorformiat und keinen unumgesetzten Propargylalkohol.

### Beispiel 4

In den Reaktionsaustrag aus Beispiel 3 werden pro Stunde 1,25 mol (70 g) Propargylalkohol, 43,7 g Toluol und 0,025 mol (3,9 g) Diisobutylformamid (=2 mol-%) so zudosiert, daß die Reaktionstemperatur in Haupt- und Nebenreaktor bei 50°C gehalten werden kann. Die Reaktion läuft für insgesamt 7 h. Der Reaktionsaustrag enthält nach GC-Analytik 65% Propargylchlorid, 0,05 % 1,3-Dichlorpropen und 0,02 % 2,3-Dichlorpropen, kein Chlorformiat und keinen unumgesetzten Propargylalkohol.

### Beispiel 5

22 g Diisobulylfonnamid (0,14 mol, 7 mol-%) werden in 70 g Toluol vorgelegt. Bei einer konstanten Temperatur von 50°C werden über 3,5 h 112 g Propargylalkohol (2 mol) und 230 g Phosgen (2,3 mol) zugefahren. Nach einer Nachreaktionszeit von 1 h bei 50°C wird nochmals Propargylalkohol zugegeben, um überschüssiges Phosgen abzureagieren. Nach einer weiteren Nachreaktionszeit von 1 h enthält der Reaktionsaustrag nach GC-Analytik 65% Propargylchlorid, 0,18 % 1,3-Dichlorpropen und 0,07 % 2,3 Dichlorpropen.

## Patentansprüche

1. Kontinuierliches Verfahren zur Herstellung von Propargylchlorid durch Umsetzung von Propargylalkohol mit einem Chlorierungsmittel in Gegenwart eines Katalysators, **dadurch gekennzeichnet, daß** das Chlorierungsmittel, Propargylalkohol und 0,1 bis 10 mol.-% des Katalysators, bezogen auf die Menge an Propargylalkohol, kontinuierlich in eine Reaktionszone dosiert und bei einer Temperatur in Bereich von 40 bis 70°C umgesetzt werden.

2. Verfahren nach Anspruch 1, **dadurch gekennzeichnet, daß** als Chlorierungsmittel Phosgen eingesetzt wird.

3. Verfahren nach Anspruch 1 oder 2, **dadurch gekennzeichnet, daß** als Katalysator Phosphinoxide, Guanidiniumsalze, offenkettige oder cyclische Alkylharnstoffe, Alkylpyridine, Alkylacetamide, N,N-Dialkylformamide oder Gemische davon eingesetzt werden.

4. Verfahren nach Anspruch 3, **dadurch gekennzeichnet, daß** als Katalysator mindestens ein Formamid der allgemeinen Formel I 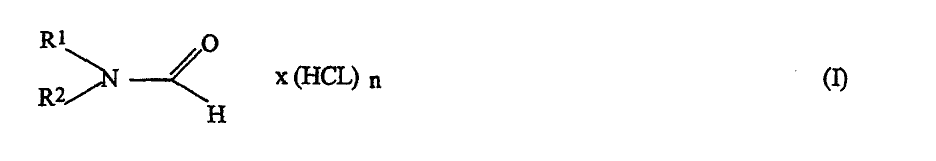 in der R¹ und R² unabhängig voneinander geradketrige oder verzweigte C₁₋₈-Alkylreste oder gemeinsam einen geradkettigen C₄₋₅-Alkyleorest bedeuten, die jeweils durch 1 bis 4 Sauerstoff- oder Stickstoffatome unterbrochen sein können, und n einen mittleren Wert im Bereich von 0 bis.3 hat, eingesetzt wird.

5. Verfahren nach einem der Ansprüche 1 bis 4, **dadurch gekennzeichnet, daß** die Umsetzung in Gegenwart von 10 bis 50 Gew.-%, bezogen auf die eingesetzte Menge an Propargylalkohol, eines inerten Verdünnungsmittels durchgeführt wird.

6. Verfahren nach Anspruch 5, **dadurch gekennzeichnet, daß** als Verdünnungsmittel ein substituierter aromatischer Kohlenwasserstoff eingesetzt wird.

7. Verfahren nach einem der Ansprüche 1 bis 6, **dadurch gekennzeichnet, daß** die Umsetzung in einer Apparatur durchgeführt wird, die einen Hauptreaktor mit nachgeschaltetem Nachreaktor, der mit einer Strippkolonne zum Strippen von nicht umgesetztem Chlorierungsmittel und gegebenenfalls Propargylchlorid versehen ist, aufweist, wobei die Dosierung der Reaktanten in den Hauptreaktor erfolgt, das Produkt aus dem Nachreaktor ausgeschleust wird und das abgestrippte Chlorierungsmittel und gegebenenfalls Propatgylchlorid nach einer Kondensation in den Haupt- und/oder Nachreaktor zurückgeführt wird.

8. Verfahren nach einem der Ansprüche 1 bis 7, **dadurch gekennzeichnet, daß** der Katalysator in einer Menge von 1 bis 5 mol.-%, bezogen auf die Menge an Propargylalkohol, eingesetzt wird.

9. Verfahren nach einem der Ansprüche 1 bis 8, **dadurch gekennzeichnet, daß** die Umsetzung bei einer Temperatur im Bereich von 50 bis 60°C erfolgt.

## Claims

1. A continuous process for preparing propargyl chloride by reacting propargyl alcohol with a chlorinating agent in the presence of a catalyst, wherein the chlorinating agent, propargyl alcohol and from 0.1 to 10 mol% of the catalyst, based on the amount of propargyl alcohol, are continuously metered into a reaction zone and reacted at from 40 to 70°C.

2. A process as claimed in claim 1, wherein the chlorinating agent used is phosgene.

3. A process as claimed in claim 1 or 2, wherein the catalyst used is a phosphine oxide, guanidinium salt, open-chain or cyclic alkylurea, alkylpyridine, alkylacetamide, N,N-dialkylformamide or a mixture thereof.

4. A process as claimed in claim 3, wherein the catalyst used is at least one formamide of the formula I 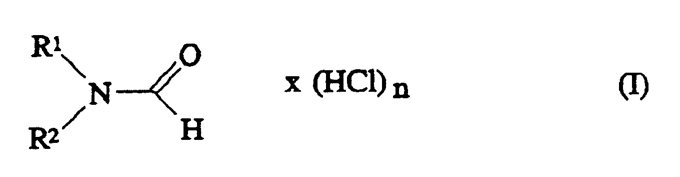 where R¹ and R² are, independently of one another, straight-chain or branched C₁-C₈-alkyl radicals or together form a straight-chain C₄-C₅-alkylene radical, each of which may be interrupted by from 1 to 4 oxygen or nitrogen atoms, and n has a mean value in the range from 0 to 3.

5. A process as claimed in any of claims 1 to 4, wherein the reaction is carried out in the presence of from 10 to 50% by weight, based on the amount of propargyl alcohol used, of an inert diluent

6. A process as claimed in claim 5, wherein the diluent used is a substituted aromatic hydrocarbon.

7. A process as claimed in any of claims 1 to 6, wherein the reaction is carried out in an apparatus which has a main reactor and a downstream after-reactor which is provided with a stripping column for stripping unreacted chlorinating agent and possibly propargyl chloride, where the reactants are metered into the main reactor, the product is discharged from the after-reactor and the stripped-out chlorinating agent and any propargyl chloride is/are condensed and returned to the main reactor and/or after-reactor.

8. A process as claimed in any of claims 1 to 7, wherein the catalyst is used in an amount of from 1 to 5 mol%, based on the amount of propargyl alcohol.

9. A process as claimed in any of claims 1 to 8, wherein the reaction is carried out at from 50 to 60°C.

## Revendications

1. Procédé continu de production de chlorure de propargyle par réaction d'alcool propargylique avec un agent de chloration en présence d'un catalyseur, **caractérisé en ce qu'**on introduit en continu dans une zone de réaction l'agent de chloration, l'alcool propargylique et 0,1 à 10 % en moles du catalyseur, par rapport à la quantité de l'alcool propargylique, et on les fait réagir à une température comprise dans la plage de 40 à 70°C.

2. Procédé selon la revendication 1, **caractérisé en ce que** l'agent de chloration utilisé est le phosgène.

3. Procédé selon la revendication 1 ou 2, **caractérisé en ce qu'**on utilise en tant que catalyseur des oxydes de phosphine, des sels de guanidinium, des alkylurées à chaîne ouverte ou cycliques, des alkylpyridines, des alkylacétamides, des N,N-dialkylformamides ou leurs mélanges.

4. Procédé selon la revendication 3, **caractérisé en ce qu'**on utilise en tant que catalyseur au moins un formamide de formule générale I 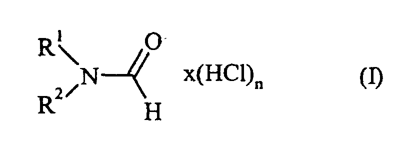 dans laquelle R¹ et R² représentent chacun indépendamment de l'autre un radical alkyle en C₁₋₈ à chaîne droite ou ramifiée ou forment ensemble un radical alkylène en C₄₋₅ à chaîne droite, chacun de ces radicaux pouvant être interrompus par 1 à 4 atomes d'oxygène ou d'azote, et n a une valeur moyenne comprise entre 0 et 3.

5. Procédé selon l'une des revendications 1 à 4, **caractérisé en ce que** la réaction est mise en oeuvre en présence de 10 à 50 % en poids, par rapport à la quantité utilisée d'alcool propargylique, d'un diluant inerte.

6. Procédé selon la revendication 5, **caractérisé en ce qu'**on utilise en tant que diluant un hydrocarbure aromatique substitué.

7. Procédé selon l'une des revendications 1 à 6, **caractérisé en ce que** la réaction est mise en oeuvre dans un appareillage, qui comporte un réacteur principal, suivi d'un réacteur en aval, lequel est pourvu d'une colonne de rectification, destinée à rectifier l'agent de chloration n'ayant pas réagi et éventuellement le chlorure de propargyle, le dosage des réactifs s'effectuant dans le réacteur principal, le produit étant évacué du réacteur en aval, et l'agent de chloration ayant subi une rectification et éventuellement le chlorure de propargyle étant, après une condensation, renvoyé dans le réacteur principal et/ou dans le réacteur en aval.

8. Procédé selon l'une des revendications 1 à 7, **caractérisé en ce que** le catalyseur est utilisé en une quantité de 1 à 5 % en moles par rapport à la quantité de l'alcool propargylique.

9. Procédé selon l'une des revendications 1 à 8, **caractérisé en ce que** la réaction est mise en oeuvre à une température comprise entre 50 et 60°C.
